# EUROPÄISCHE PATENTANMELDUNG

(11) **EP 0 933 743 A2**
(43) Veröffentlichungstag der Anmeldung: **04.08.1999**
(21) Anmeldenummer: 98123798.5
(22) Anmeldetag: 15.12.1998
(51) Int. Cl.: G08B 21/00

(54) **Vorrichtung zur Vigilanzzustandsbestimmung**

(30) Priorität: 28.01.1998 DE 19803158
(71) Anmelder: DaimlerChrysler AG, 70567 Stuttgart (DE)
(72) Erfinder: Griesinger, Manfred Dr., 71229 Leonberg (DE); Renner, Götz Dr., 73728 Esslingen (DE); Ziegler, Walter Dr., 70569 Stuttgart (DE)

(57) **Zusammenfassung**

Die Erfindung bezieht sich auf eine Vorrichtung zur Vigilanzzustandsbestimmung einer Person, insbesondere einer Fahrzeug- oder Maschinenbedienperson, mit einem Bildaufnahmesystem zum Aufnehmen von Bildern des Bereichs wenigstens eines Auges der Person und einem Bildauswertesystem, das die vom Bildaufnahmesystem aufgenommenen Bilder auswertet und Lidschlußerkennungsmittel beinhaltet.

Erfindungsgemäß weist das Bildauswertesystem Mittel zur Pupillendurchmesserbestimmung und eine Auswerteeinrichtung auf, die den Vigilanzzustand in Abhängigkeit von der durch die Lidschlußerkennungsmittel erhaltenen Lidschlußzustandsinformation und von der durch die Pupillendurchmesserbestimmungsmittel erhaltenen Pupillendurchmesserzustandsinformation unter Einklassifizieren in jeweils eine von mindestens drei Stufen bestimmt.

Verwendung z.B. zur Bestimmung des Vigilanzzustands von Kraftfahrzeugführern.

## Beschreibung

Die Erfindung bezieht sich auf eine Vorrichtung zur Vigilanzzustandsbestimmung bei einer Person, insbesondere einer Fahrzeug- oder Maschinenbedienperson, nach dem Oberbegriff des Anspruchs 1. Derartige Vorrichtungen dienen beispielsweise dazu, den Vigilanzzustand eines Fahrzeugführers kontinuierlich zu überwachen und ihn dadurch während des Fahrens rechtzeitig vor dem Einschlafen zu warnen. Dazu werden vom Bildaufnahmesystem Bilder des Bereichs wenigstens eines Auges aufgenommen und von einem nachgeschalteten Bildauswertesystem analysiert.

Bei Vorrichtungen dieser Art, wie sie auch aus den Offenlegungsschriften DE 196 21 435 A1 und DE 197 15 519 A1 sowie den Patentschriften US 4.359.724, US 5.402.109 und US 5.566.067 bekannt sind, beinhaltet das Bildauswertesystem Mittel zur Lidschlußerkennung, mit denen ein zunehmendes zeitweises Schließen der Augen festgestellt werden kann, was als zunehmende Müdigkeit interpretiert wird. Beim System der DE 196 21 435 A1 kann zudem die Fähigkeit vorgesehen sein, die Pupillenbewegung, speziell die Geschwindigkeit der Pupille, zu messen und dies als weiteren Parameter für die Entscheidung heranzuziehen, ob die überwachte Person wach oder schläfrig ist. Beim System der DE 197 15 519 A1, mit dem der Schläfrigkeitsgrad eines Fahrzeugführers geschätzt werden soll, wird in einer ersten Zeitspanne nach dem Starten des Fahrzeugs eine Häufigkeitsverteilung der Blinzeldauer des Fahrers erstellt und daraus ein Schwellenwert abgeleitet, der dann in einer anschließenden zweiten Zeitspanne zur Beurteilung des Schläfrigkeitsgrades herangezogen wird. Diese Vorgehensweise soll den Unterschieden in der Blinzeldauer und Blinzelhäufigkeit zwischen verschiedenen Personen Rechnung tragen, wobei vorausgesetzt wird, daß der Fahrer in der Anfangsphase nach dem Starten des Fahrzeugs einen niedrigen Schläfrigkeitsgrad besitzt.

Aus der medizinischen Pupillographie sind Bildaufnahme- und Bildauswertesysteme mit Mitteln zur Bestimmung des Pupillendurchmessers und/oder der Augenbewegung, d.h. der Pupillenbewegung bekannt, um damit während Augenuntersuchungen den Pupillendurchmesser bzw. die Bewegungen des untersuchten Auges zu erfassen. Systeme dieser Art sind in den Offenlegungsschfiften DE 35 41 726 A1, DE 44 19 489 A1 und DE 41 40 160 A1 sowie den Patent-schriften US 3.966. 310, US 4.755.043, US 4.850.691, US 5.187.506 und US 5.204.703 beschrieben.

Es ist aus der Literatur des weiteren bekannt, daß am Verhalten des Pupillendurchmessers eine Übermüdung festgestellt werden kann. Speziell zeigen Untersuchungen, daß mit zunehmender Müdigkeit die Pupillen sich zunächst verengen, selbst wenn die Umgebungshelligkeit nicht besonders hoch ist, und anschließend Schwingungen des Pupillendurchmessers auftreten, was den Einsatz der Pupillographie zur Identifizierung einschlafgefährdeter Personen, z.B. im Straßen-, See- und Luftverkehr, möglich erscheinen läßt, siehe B. Wilhelm, H. Wilhelm, "Die Pupille als Schlaf-Wach-Indikator", Z. prakt. Augenheilkd. 15, 1994, Seite 185 und I.E. Loewenfeld, "The Pupil", Band I, Wayne State University Press, Detroit, 1993.

Der Erfindung liegt als technisches Problem die Bereitstellung einer Vorrichtung zur Vigilanzzustandsbestimmung der eingangs genannten Art zugrunde, mit welcher der Wachheits- bzw. Vigilanzzustand einer Person, insbesondere eines Fahrzeugführers oder Maschinenbedieners, besonders zuverlässig bestimmt werden kann, um daraus geeignete Folgerungen zu ziehen, z.B. Abgabe einer rechtzeitigen Warnmeldung oder Verbringung eines von der Person zu steuernden bzw. bedienenden Systems in einen sicherheitsunkritischen Zustand.

Die Erfindung löst dieses Problem durch die Bereitstellung einer Vigilanzzustandsbestimmungsvorrichtung mit den Merkmalen des Anspruchs 1. Bei dieser Vorrichtung weist deren Bildauswertesystem neben Lidschlußerkennungsmitteln Mittel zur Pupillendurchmesserbestimmung sowie eine Auswerteeinrichtung auf, die den Vigilanzzustand in Abhängigkeit von der durch die Lidschlußerkennungsmittel erhaltenen Lidschlußzustandsinformation und von der durch die Pupillendurchmesserbestimmungsmittel erhaltenen Pupillendurchmesserzustandsinformation bestimmt, wobei der Vigilanzzustand charakteristischerweise jeweils in eine von mindestens drei Stufen einklassifiziert wird. Durch das Vorhandensein sowohl der Lidschlußerkennungsmittel als auch der Pupillendurchmesserbestimmungsmittel wird eine gewisse Redundanz sowie eine hohe Zuverlässigkeit der Vigilanzzustandsbestimmung erzielt. Das Einklassifizieren des Vigilanzzustands in drei oder mehr Stufen unterschiedlicher Vigilanz schafft die Möglichkeit, abgestuft auf eine beginnende Übermüdigung der überwachten Person reagieren zu können.

Bei einer nach Anspruch 2 weitergebildeten Vorrichtung enthält das Bildauswertesystem Mittel zur Bestimmung der Umgebungshelligkeit, und die Auswerteeinrichtung besitzt eine Korrelationseinheit, mit welcher festgestellt werden kann, in welchem Grad der Pupillendurchmesser mit der Umgebungshelligkeit korreliert ist. Auf diese Weise können Änderungen des Pupillendurchmessers, die auf Schwankungen der Umgebungshelligkeit beruhen, von vigilanzbedingten Pupillendurchmesserschwankungen sicher unterschieden werden.

Eine nach Anspruch 3 weitergebildete Vorrichtung ist so ausgelegt, daß sie bei der mehrstufigen Vigilanzzustandsbestimmung auf die höchste Vigilanzstufe, d.h. auf einen Wachzustand, schließt, wenn dies sowohl durch kurze Lidschlußdauern und vergleichsweise hohe Lidschlußfrequenzen als auch durch Ausbleiben von nicht mit dem Umgebungslicht korrelierten Pupillendurchmesserschwankungen indiziert ist. Hingegen wird das Vorliegen des niedrigsten Vigilanzzustands, d.h. des höchsten Müdigkeitsgrades, angenommen, wenn die beobachteten Lidschlußdauern übermäßig hoch sind.

Eine nach Anspruch 4 weitergebildete Vorrichtung ermöglicht eine mindestens vierstufige Vigilanzzustandsbestimmung, wobei neben den Stufen höchster und geringster Vigilanz eine erste Zwischenstufe höherer und eine zweite Zwischenstufe niedrigerer Vigilanz dadurch definiert sind, daß gemessene Pupillendurchmesserschwingungen im einen Fall unterhalb und im anderen Fall oberhalb eines entsprechenden Schwellwertes liegen und außerdem wenigstens eine der Bedingungen erfüllt ist, daß die Pupillendurchmesserschwankungen nicht gut mit Umgebungslichtschwankungen korrelieren oder die Lidschlußdauer in einem mittleren Bereich liegt oder die Lidschlußfrequenz unterhalb eines entsprechenden Schwellwertes liegt. Dies realisiert eine sehr feinfühlig abgestufte Müdigkeitserkennung.

Eine nach Anspruch 5 weitergebildete Vorrichtung beinhaltet eine vom Bildauswertesystem angesteuerte Warneinheit, die in Abhängigkeit vom Vigilanzzustand entsprechende Warnsignale lokal, insbesondere für die überwachte Person, oder auch als fernübertragenes Signal an eine von der überwachten Person räumlich entfernte Stelle, z.B. im Fall eines Fahrzeuges an eine Zentrale oder andere Fahrzeuge, abgibt.

Bei einer nach Anspruch 6 weitergebildeten Vorrichtung ist eine Warneinheit vorgesehen, die eine Fahrzeug- bzw. Maschinensteuerungseinrichtung in Abhängigkeit vom erfaßten Vigilanzzustand derart ansteuert, daß bei Erkennen einer entsprechend niedrigen Vigilanz der überwachten Person das von dieser gesteuerte Fahrzeug bzw. die von dieser gesteuerte Maschine von der Steuerungseinrichtung in einen sicheren Betriebszustand gebracht wird. Dies beugt z.B. bei Fahrzeugen Unfallgefahren aufgrund einer Übermüdung des Fahrers aktiv dadurch vor, daß das Fahrzeug von einem durch die Warneinheit angesprochenen, autonomen Fahrzeugführungssystem auf sichere Weise verlangsamt oder ganz zum Halten gebracht wird.

Bei einer nach Anspruch 7 weitergebildeten Vorrichtung ist eine Bedieneinheit vorgesehen, die auf die Erkennung einer entsprechend niedrigen Vigilanz hin von einer Warneinheit dazu veranlaßt wird, der überwachten Person eine Vigilanztestaufgabe zu stellen, von deren ordnungsgemäßer Erledigung dann weitere Systemaktionen abhängig gemacht werden können.

Eine vorteilhafte Ausführungsform der Erfindung ist in den Zeichnungen dargestellt und wird nachfolgend beschrieben. Hierbei zeigen:
- Fig. 1: eine schematische Seitenansicht eines einer überwachten Person zugeordneten Bildaufnahmesystems einer Vigilanzzustandsbestimmungsvorrichtung,
- Fig. 2: eine schematische Darstellung des Bildrahmens des Bildaufnahmesystems von Fig. 1 und
- Fig. 3: eine Blockdiagrammdarstellung der zum Bildaufnahmesystem gemäß Fig. 1 gehörigen Vigilanzzustandsbestimmungsvorrichtung.

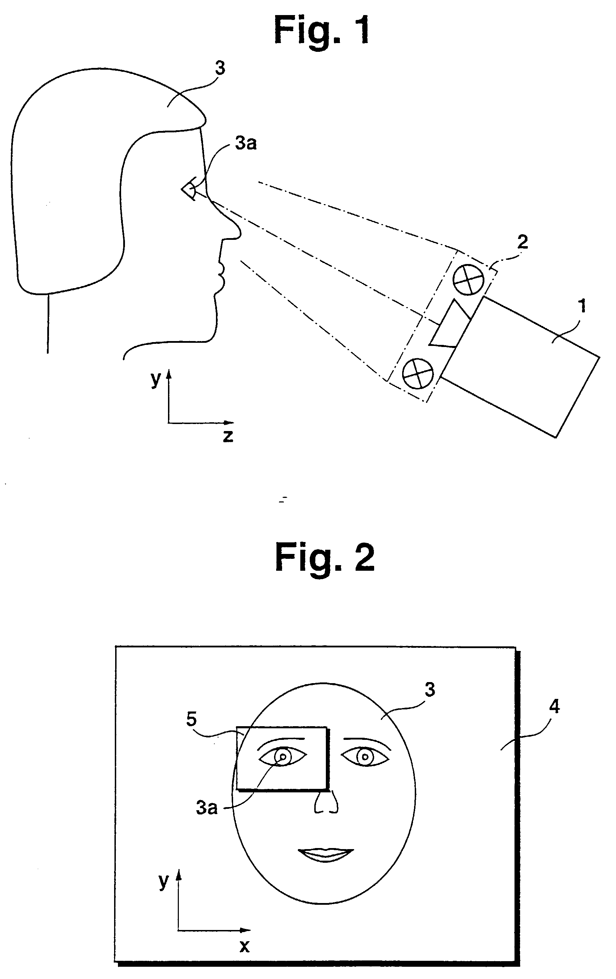

Wie in den Figuren 1 und 2 dargestellt, weist die gezeigte Vigilanzzustandsbestimmungsvorrichtung ein optisches Bildaufnahmesystem in Form einer Kamera 1 und einer Infrarot-Beleuchtungseinrichtung 2 auf. Die Beleuchtungseinrichtung 2 umgibt möglichst dicht die optische Achse der Kamera 1 und dient dazu, den Netzhauthintergrund eines überwachten Auges 3a einer Fahrzeug- oder Maschinenbedienperson 3 möglichst hell und unabhängig von umgebendem Fremdlicht im Bild des Auges 3a hervortreten zu lassen. Der Fokus von Kamera 1 und Beleuchtungseinrichtung 2 ist so gewählt, daß das beobachtete Auge 3a, im gezeigten Beispiel das rechte Auge, auch bei Berücksichtigung einer gewissen notwendigen Bewegungsfreiheit des Kopfes der Person 3 in der xy-Ebene eines angedeuteten xyz-Koordinatensystems stets im Kamerablickfeld 4 erscheint und ein Beobachtungsfenster 5 des Bildaufnahmesystems auf ein lichtempfindliches CCD-Array der als CCD-Videokamera 1 ausgelegten Kamera fällt. Des weiteren sind Blende und Fokus der Kamera 1 so gewählt, daß auch bei einer gewissen notwendigen Bewegungsfreiheit des Kopfes der Person 3 in z-Richtung das Auge 3a stets scharf auf das lichtempfindliche CCD-Array abgebildet wird. Auf diese Weise ist das Bildaufnahmesystem in der Lage, bei Tag und Nacht, d.h. unabhängig vom Umgebungslicht, das Auge 3a der Fahrzeug- oder Maschinenbedienperson 3 berührungsfrei und abstandsunabhängig zu überwachen.

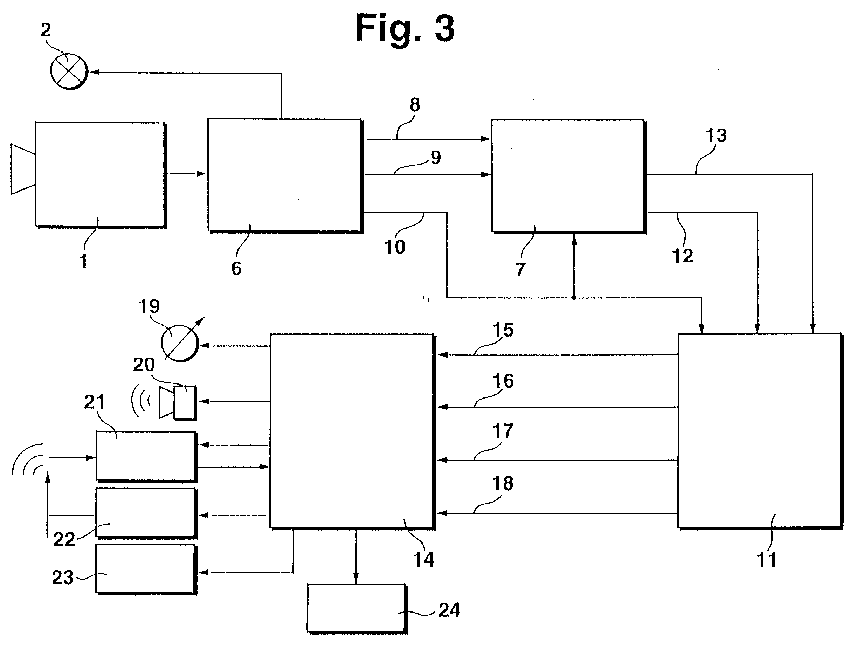

Fig. 3 zeigt die gesamte Vigilanzzustandsbestimmungsvorrichtung in Blockdiagrammform. Wie daraus ersichtlich, ist bei dieser Vorrichtung dem Bildaufnahmesystem 1, 2 ein Bildauswertesystem nachgeschaltet, das eingangsseitig eine Bildverarbeitungseinheit 6 aufweist, der die von der Kamera 1 aufgenommenen Bilder übermittelt werden und die daraus nach einem bekannten Bildverarbeitungsverfahren das überwachte Auge erkennt und eine Nachführung des Beobachtungsfensters 5 bewerkstelligt. Die Bildverarbeitungseinheit 6 arbeitet mit zwei Betriebszuständen, zwischen denen ständig hin und her geschaltet wird. In einem ersten Betriebszustand wird bei ausgeschalteter Infrarotbeleuchtung 2 die durch das Umgebungslicht, d.h. Fremdlicht, hervorgerufene mittlere Helligkeit im Beobachtungsfenster 5 gemessen und diese Information als ein Helligkeitssignal 8 abgegeben, ohne daß in diesem Betriebszustand eine weitere Bildauswertung erfolgt. In einem zweiten Betriebszustand ist die Infrarotbeleuchtung 2 eingeschaltet, und die Bildverarbeitungseinheit 6 wertet das vom überwachten Auge erhaltene Bild nach folgenden Kriterien aus.

Zum einen erfaßt sie durch entsprechende herkömmliche Pupillendurchmesserbestimmungsmittel, wie sie z.B. aus der oben erwähnten DE 44 19 489 A1 bekannt sind, den Pupillendurchmesser des beobachteten Auges. Wie oben erwähnt, verringert sich der Pupillendurchmesser mit zunehmender Müdigkeit, und unmittelbar vor dem Einschlafen treten Schwingungen des Pupillendurchmessers im Bereich von 0,1Hz bis 1Hz auf. Die Information über den momentanen Pupillendurchmesser gibt die Bildauswerteeinheit 6 in Form eines Pupillendurchmessersignals 9 ab. Zum anderen besitzt die Bildverarbeitungseinheit 6 herkömmliche Lidschlußerkennungsmittel, mit denen die Lidschlußdauer und die Lidschlußfrequenz für das kontinuierlich überwachte Auge ermittelt werden. Die Bildverarbeitungseinheit 6 gibt ein entsprechendes Lidschlußsignal 10 ab, das einerseits eine Information über die Zeitabschnitte, in denen das Auge geschlossen ist und somit kein Pupillendurchmesser bestimmt werden kann, und andererseits über die Zeitdauer und die Frequenz des Lidschlusses enthält, die bekanntermaßen ebenfalls ein Maß für die Müdigkeit der überwachten Person 3 sind.

Des weiteren weist das Bildauswertesystem eine der Bildverarbeitungseinheit 6 nachgeschaltete Auswerteeinrichtung auf, die aus einer Korrelationseinheit 7 und einer nachgeschalteten Klassifikationseinheit 11 besteht. Die Korrelationseinheit 7 korreliert den zeitlichen Verlauf des kontinuierlich gemessenen Pupillendurchmessers, wie er sich aus dem ihr zugeführten Pupillendurchmessersignal 9 ergibt, mit der über das zugeführte Umgebungshelligkeitssignal 8 erhaltenen Information über die Umgebungshelligkeit, wodurch sie in der Lage ist, die normale Pupillenreaktion auf Schwankungen der Umgebungshelligkeit als Störeinfluß zu eliminieren. Die Korrelationseinheit 7 gibt ein entsprechendes Korrelationssignal 12 ab, das die Information darüber enthält, inwieweit Änderungen des Pupillendurchmessers von einer sich ändernden Umgebungshelligkeit verursacht sind oder nicht. Außerdem erzeugt die Korrelationseinheit 7 durch eine Filterung des Pupillendurchmessersignals 9 im Bereich von 0,1Hz bis 1Hz ein Pupillendurchmesserschwingungssignal 13, das eine Information darüber enthält, ob im genannten Frequenzbereich Schwingungen des Pupillendurchmessers unabhängig vom Umgebungslicht auftreten oder nicht. Der Korrelations- und Filterungsprozeß in der Korrelationseinheit 7 wird vom zugeführten Lidschlußsignal 10 gesteuert, d.h. unterbrochen, wenn das Auge geschlossen ist, und fortgeführt, wenn das Auge offen ist.

Die Klassifikationseinheit 11 führt anhand des Lidschlußsignals 10, des Korrelationssignals 12 und des Pupillendurchmesserschwingungssignals 13, die ihm als Eingangssignale zugeführt werden, eine Klassifikation durch, die den Vigilanzzustand der Fahrzeug- oder Maschinenbedienperson 3 in vier verschiedene Stufen einteilt und für jede Stufe ein charakteristisches Ausgangssignal 15, 16, 17, 18 abgibt.

Ein erstes Ausgangssignal 15 der Klassifikationseinheit 11 ist einer ersten Stufe höchster Vigilanz zugeordnet, die den Normalzustand, d.h. den "Wach"-Zustand der Person 3 darstellt. Auf diese Vigilanzstufe schließt die Klassifikationseinheit 11 dann, wenn die folgenden vier Bedingungen erfüllt sind. Die erste Bedingung ist, daß das Pupillendurchmesserschwingungssignal 13 unterhalb eines vorgebbaren Pupillendurchmesserschwingungs-Schwellwertes liegt, d.h. daß keine merklichen Schwingungen des Pupillendurchmessers beobachtet werden, die nicht durch das Umgebungslicht verursacht sind. Die zweite Bedingung ist, daß das Korrelationssignal 12 eine gute Korrelation des Pupillendurchmessers mit dem Umgebungslicht anzeigt. Die dritte Bedingung ist, daß die aus dem Lidschlußsignal 10 erkannte Lidschlußdauer unterhalb eines ersten vorgebbaren Lidschlußdauer-Schwellwertes liegt, der für den Wachzustand eines Menschen kennzeichnend ist, und die vierte Bedingung ist, daß die ebenfalls aus dem Lidschlußsignal 10 erhaltene Lidschlußfrequenz oberhalb eines vorgebbaren Lidschlußfrequenz-Schwellwertes liegt, der ebenfalls kennzeichnend für den Wachzustand eines Menschen ist.

Ein zweites Ausgangssignal 16-der Klassifikationseinheit 11 repräsentiert einen "Müde"-Zustand als einer Stufe zweithöchster Vigilanz. Auf diese Vigilanzstufe schließt die Klassifikationseinheit 11, wenn das Pupillendurchmesserschwingungssignal 13 unterhalb des Pupillendurchmesserschwingungs-Schwellwertes liegt und zusätzlich wenigstens eine der folgenden drei Bedingungen erfüllt ist, daß nämlich das Korrelationssignal 12 eine schlechte Korrelation des Pupillendurchmessers mit dem Umgebungslicht anzeigt oder, daß die Lidschlußdauer oberhalb des ersten Lidschlußdauer-Schwellwertes und unterhalb eines zweiten vorgebbaren Lidschlußdauer-Schwellwertes liegt, der anzeigt, daß das Auge ungewöhnlich lange geschlossen bleibt, oder daß die Lidschlußfrequenz unterhalb des Lidschlußfrequenz-Schwellwertes liegt.

Ein drittes Ausgangssignal 17 der Korrelationseinheit 11 charakterisiert eine Stufe zweitniedrigster Vigilanz, die einen "Einschlafgefährdet"-Zustand bedeutet. Auf diesen Zustand erkennt die Klassifikationseinheit 11 dann, wenn das Pupillendurchmesserschwingungssignal 13 oberhalb des Pupillendurchmesserschwingungs-Schwellwertes liegt und wenigstens eine der drei folgenden Bedingungen erfüllt ist, daß nämlich das Korrelationssignal eine schlechte Korrelation des Pupillendurchmessers mit dem Umgebungslicht anzeigt oder daß die Lidschlußdauer oberhalb des ersten Lidschlußdauer-Schwellwertes und unterhalb des zweiten Lidschlußdauer-Schwellwertes liegt oder daß die Lidschlußfrequenz unterhalb des Lidschlußfrequenz-Schwellwertes liegt.

Ein viertes Ausgangssignal 18 der Klassifikationseinheit 11 entspricht der Stufe niedrigster Vigilanz, die einem "Augen-zu"-Zustand entspricht. Auf diesen Zustand erkennt die Klassifikationseinheit 11 dann, wenn die Lidschlußdauer oberhalb des zweiten Lidschlußdauer-Schwellwertes liegt.

Dem Bildauswertesystem ist eine Warneinheit 14 nachgeschaltet, der die vier Ausgangssignale 15 bis 18 der Klassifikationseinheit 11 zugeführt werden und die dadurch die Information über den momenten Vigilanzzustand erhält. Die Warneinheit 14 führt nun in Abhängigkeit von der jeweils vorliegenden Vigilanzstufe bestimmte Aktionen durch, um die Fahrzeug- oder Maschinenbedienperson 3 wieder in einen Zustand höherer Vigilanz zu bringen oder sie bzw. Außenstehende rechtzeitig zu warnen oder das Fahrzeug bzw. die Maschine in einen sicheren, ungefährlichen Betriebszustand zu überführen.

So ist eine optische Anzeige 19 vorgesehen, über welche die Warneinheit 14 der überwachten Person 3 den Grad ihrer Müdigkeit bzw. Vigilanz anzuzeigen vermag. Außerdem ist ein akustischer Alarmgeber 20 vorgesehen, über den die Warneinheit 14 die Person 3 bei auftretender Müdigkeit warnen kann. Des weiteren ist eine Bedieneinheit 21 vorgesehen, die von der Warneinheit 14 dazu angesteuert werden kann, der Person 3 eine Nebenaufgabe zwecks Vigilanztest abzuverlangen, über deren Erledigung die Bedieneinheit 21 der Warneinheit 14 eine Rückmeldung gibt, wonach die Warneinheit 14 weitere Aktionen in Abhängigkeit von der Erledigung dieser Nebenaufgabe abhängig macht. Zusätzlich ist eine Sendeeinrichtung 22 vorgesehen, über welche die Warneinheit 14 den festgestellten Vigilanzzustand an eine Zentrale oder z.B. an andere Fahrzeuge zur Warnung mitteilen kann. Eine Speichereinrichtung 24 kann von der Warneinheit 14 dazu verwendet werden, den festgestellten Vigilanzzustand der Person 3 für eine bestimmte zurückliegende Zeitdauer dauerhaft und auslesbar aufzuzeichnen. Zusätzlich ist vorgesehen, daß die Warneinheit 14 eine atuomatische Fahrzeug- bzw. Maschinensteuerung 23 so anzusteuern vermag, daß diese bei Bedarf das Fahrzeug bzw. die Maschine selbsttätig in einen sicheren, ungefährlichen Betriebszustand überführt. Dies wird von der Warneinheit 14 inbesondere dann veranlaßt, wenn ihr der "Augen-zu"-Zustand niedrigster Vigilanz von der Klassifikationseinheit 11 übermittelt wird oder wenn die an der Bedieneinrichtung 21 gestellte Nebenaufgabe von der überwachten Person 3 unzureichend oder gar nicht gelöst wurde.

Die obige Beschreibung eines vorteilhaften Ausführungsbeispiels zeigt, daß die erfindungsgemäße Vorrichtung eine zuverlässige Vigilanzzustandsbestimmung durch kombinierte Auswertung einer Lidschlußinformation und einer Information über auftretende Pupillendurchmesserschwingungen ermöglicht. Es versteht sich, daß die erfindungsgemäße Vorrichtung nicht auf dieses Beispiel beschränkt ist, sondern weitere Realisierungen umfaßt. So kann neben dem beschriebenen, mit Infrarotstrahlung arbeitenden Bildaufnahmesystem ein System eingesetzt werden, das mit einer anderen Strahlung arbeitet, wobei diese zweckmäßigerweise außerhalb des sichtbaren Spektrums gewählt wird. Des weiteren können je nach Anwendungsfall eine oder mehrere der von der gezeigten Warneinheit 14 angesteuerten Komponenten 19 bis 24 und ggf. auch die Warneinheit 14 entfallen. Die Vigilanzzustandsbestimmung kann anwendungsfallabhängig unter Benutzung modifzierter Bedingungen auch nur dreistufig oder in mehr als vier Stufen erfolgen.

## Patentansprüche

1. Vorrichtung zur Vigilanzzustandsbestimmung bei einer Person, insbesondere einer Fahrzeug- oder Maschinenbedienperson, mit
- einem Bildaufnahmesystem (1, 2) zum Aufnehmen von Bildern des Bereiches (5) wenigstens eines Auges (3a) der Person (3) und
- einem Bildauswertesystem (6, 7, 11), das zum Auswerten der vom Bildaufnahmesystem aufgenommenen Bilder dient und Lidschlußerkennungsmittel beinhaltet,
**dadurch gekennzeichnet, daß**
- das Bildauswertesystem (6, 7, 11) Pupillendurchmesserbestimmungsmittel und eine Auswerteeinrichtung aufweist, die den Vigilanzzustand in Abhängigkeit von der durch die Lidschlußerkennungsmittel erhaltenen Lidschlußzustandsinformation und von der durch die Pupillendurchmesserbestimmungsmittel erhaltenen Pupillendurchmesserzustandsinformation unter Einklassifizieren in jeweils eine von mindestens drei Vigilanzstufen bestimmt.

2. Vorrichtung nach Anspruch 1, weiter
**dadurch gekennzeichnet, daß**
- das Bildauswertesystem (6, 7, 11) Mittel zur Bestimmung der Umgebungshelligkeit umfaßt und
- die Auswerteeinrichtung eine Korrelationseinheit (7) aufweist, welche den Korrelationsgrad der Pupillendurchmesserzustandsinformation mit der durch die Umgebungshelligkeitsbestimmungsmittel erhaltenen Umgebungshelligkeitsinformation bestimmt, und den Vigilanzzustand zusätzlich in Abhängigkeit von der durch die Korrelationseinheit erhaltenen Korrelationsgradinformation bestimmt.

3. Vorrichtung nach Anspruch 2, weiter
**dadurch gekennzeichnet, daß**
- die Lidschlußzustandsinformation eine Lidschlußdauerinformation und eine Lidschlußfrequenzinformation umfaßt und
- von der Auswerteeinrichtung die höchste Vigilanzstufe angegeben wird, wenn die Lidschlußdauer unterhalb eines vorgebbaren ersten Lidschlußdauer-Schwellwertes liegt, die Lidschlußfrequenz oberhalb eines vorgebbaren Lidschlußfrequenz-Schwellwertes liegt und der Grad an nicht von der Umgebungshelligkeit verursachten Pupillendurchmesserschwingungen unterhalb eines vorgebbaren Pupillendurchmesserschwingungs-Schwellwertes liegt, und die niedrigste Vigilanzstufe angegeben wird, wenn die Lidschlußdauer oberhalb eines vorgebbaren zweiten Lidschlußdauer-Schwellwertes liegt.

4. Vorrichtung nach Anspruch 3, weiter
**dadurch gekennzeichnet, daß**
- von der Auswerteeinrichtung (2) Vigilanzzwischenstufen angegeben werden, von denen eine höhere Zwischenstufe angegeben wird, wenn der Grad an nicht von der Umgebungshelligkeit verursachten Pupillendurchmesserschwingungen unterhalb des vorgebbaren Pupillendurchmesserschwingungs-Schwellwertes liegt und entweder die Lidschlußdauer zwischen dem ersten Lidschlußdauer-Schwellwert und dem demgegenüber größeren, zweiten Lidschlußdauer-Schwellwert liegt oder die Lidschlußfrequenz unterhalb des Lidschlußfrequenz-Schwellwertes liegt, während die andere Zwischenstufe niedrigerer Vigilanz angegeben wird, wenn der Grad an nicht von der Umgebungshelligkeit verursachten Pupillendurchmesserschwingungen oberhalb des Pupillendurchmesserschwingungs-Schwellwertes liegt und entweder die Lidschlußdauer zwischen dem ersten und dem zweiten Lidschlußdauer-Schwellwert liegt oder die Lidschlußfrequenz unterhalb des Lidschlußfrequenz-Schwellwertes liegt.

5. Vorrichtung nach einem der Ansprüche 1 bis 4, weiter
**gekennzeichnet durch**
eine vom Bildauswertesystem angesteuerte Warneinheit (14), die in Abhängigkeit vom festgestellten Vigilanzzustand Warnmeldungen lokal und/oder fernreichweitig abgibt.

6. Vorrichtung nach einem der Ansprüche 1 bis 5, weiter
**gekennzeichnet durch**
eine vom Bildauswertesystem angesteuerte Warneinheit (14), die in Abhängigkeit vom festgestellten Vigilanzzustand eine Fahrzeug- oder Maschinensteuerung (23) ansteuert, mit der bei entsprechend niedriger festgestellter Vigilanz das Fahrzeug bzw. die Maschine in einen sicheren Betriebszustand gebracht wird.

7. Vorrichtung nach einem der Ansprüche 1 bis 6, weiter
**gekennzeichnet durch**
eine vom Bildauswertesystem angesteuerte Warneinheit (14) und eine Bedieneinheit (21), an der die Warneinheit (14) bei entsprechend niedriger festgestellter Vigilanz die Durchführung einer Vigilanztestaufgabe durch die überwachte Person (3) anfordert.
